# EUROPEAN PATENT APPLICATION

(11) **EP 2 508 155 A1**
(43) Date of publication of application: **10.10.2012**
(21) Application number: 10833107.5
(22) Date of filing: 17.11.2010
(51) Int. Cl.: A61F 13/15, A61F 13/472, A61F 13/511

(54) **BODILY FLUID TREATMENT ARTICLE**

(30) Priority: 30.11.2009 JP 2009272385
(71) Applicant: Unicharm Corporation, Ehime 799-0111 (JP)
(72) Inventor: SUGA, Ayami, Kanonji-shi Kagawa 769-1602 (JP)
(74) Representative: Eke, Philippa Dianne
(86) International application number: PCT/JP2010/070432
(87) International publication number: WO 2011/065260

(57) **Abstract**

The present invention provides a bodily fluid absorbent article having visually recognizable display elements formed of coloring material wherein an average mass of coloring material per a predetermined area gradually increases from inside towards outside as viewed in a longitudinal direction. A panty liner 1 includes a liquid-pervious topsheet 2 lying on a skin-facing side, a liquid-impervious backsheet 3 lying on a garment-facing side and a second sheet 4 sandwiched between these sheets 2, 3 as an intermediate layer. The panty liner 1 is formed with display elements 5 adapted to be visually recognized through the topsheet 2. The display elements 5 are coated with coloring material in a gradation such that a density of the coloring material gradually increases outwardly from an imaginary transverse center line Q-Q. Specifically, the density of the coloring material in the respective display elements 5 gradually increases outwardly.

## Description

### {Technical Field}

The present invention relates to bodily fluid absorbent articles such as menstruation napkins, urine absorbent pads, panty liners or the like.

### {Background}

Bodily fluid absorbent articles such as menstruation napkins characterized by being locally colored are known. For example, PTL 1 discloses a bodily fluid absorbent article including an absorbent core sandwiched between a topsheet and a backsheet and having a top surface and bottom surface. This bodily fluid absorbent article of the prior art has a colored region and a non-colored region wherein the colored region can be seen through the topsheet from the surface thereof. By forming such colored region, it is possible to make menstrual blood which has soiled the napkin less noticeable.

### {Citation List}

### {Patent Literature}

{PTL 1} JP 2005-512682 A

### {Summary}

### {Technical Problem}

According to the disclosure of PTL 1, the colored region is in the middle of the wearing article in a longitudinal direction as well as in a transverse direction. Such colored region uses at least a light color and a dark color to create a shade and thereby to make absorbed menstrual blood less noticeable. However, the description concerning the formation of the colored region is limited to utilization of light and dark colors and no description of the other concepts or ideas can be found.

An object of the present invention is to provide a bodily fluid absorbent article having visually recognizable display elements formed by coloring material wherein an average mass of coloring material per a predetermined area gradually increases outward as viewed in a longitudinal direction.

### {Solution to Problem}

According to the present invention, there is provided a bodily fluid absorbent article having a longitudinal direction, a transverse direction, a skin-facing side and a garment-facing side and including a liquid-pervious topsheet lying on the skin-facing side, a liquid-impervious backsheet lying on the garment-facing side and an intermediate layer sandwiched between the top- and backsheets.

In the bodily fluid absorbent article, the feature of the present invention resides in that display elements are formed of coloring material applied to at least one of the topsheet and the intermediate layer so that an average mass of coloring material gradually increases outwardly as viewed in the longitudinal direction. The term "intermediate layer" used herein includes, for example, a liquid-pervious fibrous nonwoven fabric sheet, a liquid-absorbent fibrous nonwoven fabric sheet and an absorbent member including fluff pulp fibers and the like.

According to one embodiment of the present invention, an average mass of coloring material per a predetermined area gradually increases from an imaginary longitudinal center line bisecting a width dimension in the transverse direction outwardly as seen in the transverse direction.

According to another embodiment of the present invention, a non-display region including none of the display elements is defined in a middle region of the article as viewed in the longitudinal direction.

According to still another embodiment of the present invention, the display elements are formed of a plurality of graphics and the graphics lying closer to an outer edge of the article are larger than the graphics lying closer to the middle of the article as viewed in at least one of the longitudinal direction and the transverse direction. It should be understood here that the term "predetermined area" used herein corresponds to (a full width dimension of the bodily fluid absorbent article in the transverse direction) x (a predetermined dimension of the bodily fluid absorbent article in the longitudinal direction). When a relatively large graphic is located closer to the outer periphery of the article and a relatively small graphic is located closer to the middle of the article as viewed in the longitudinal direction of the article, an average mass of coloring material per the predetermined area is larger in the region of the larger graphic than in the region of the smaller graphic.

According to yet another embodiment of the present invention, a density of coloring material is higher in the graphic lying closer to an outer edge of the article than in the graphic lying closer to the middle of the article as viewed in at least one of the longitudinal direction and the transverse direction. The term "predetermined area" may be construed also to be the predetermined area in the respective graphics. As a consequence of comparing a density of coloring material in the graphic lying closer to the outer periphery of the article with a density of coloring material in the graphic lying closer to the middle of the article, the density of coloring material should be higher in the graphic lying closer to the outer periphery of the article than in the graphic lying closer to the middle of the article.

### {Advantageous Effects of Invention}

The visually recognizable display elements are formed of coloring material applied to at least one of the topsheet and the intermediate layer so that an average mass of coloring material gradually increases outwardly as viewed in the longitudinal direction of the article. With such an arrangement, the average mass of coloring material applied to the topsheet or the intermediate layer gradually decreases inwardly as viewed in the longitudinal direction of the article and therefore absorption of bodily fluids would not be counteracted by the presence of coloring material.

### {Brief Description of Drawings}

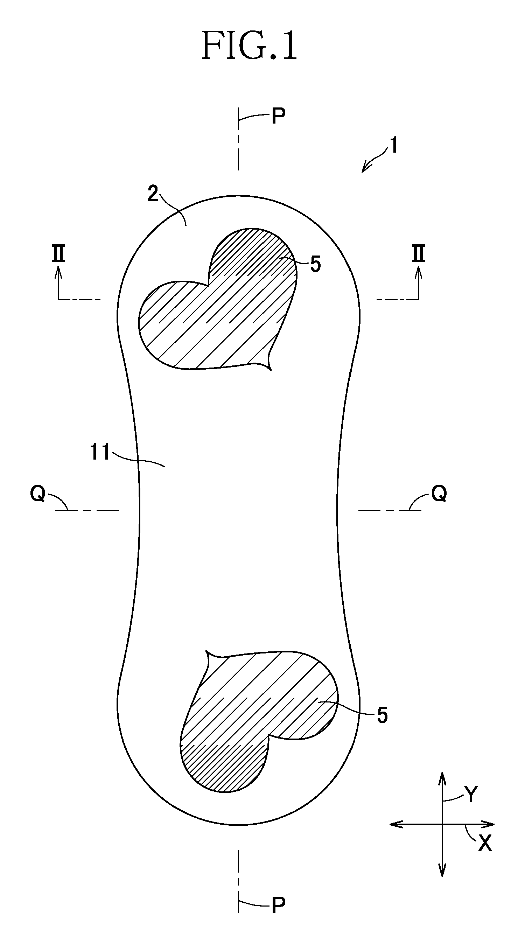

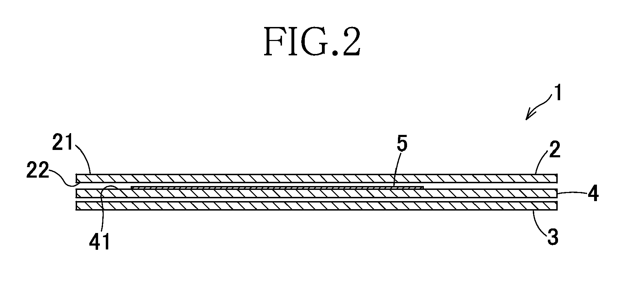

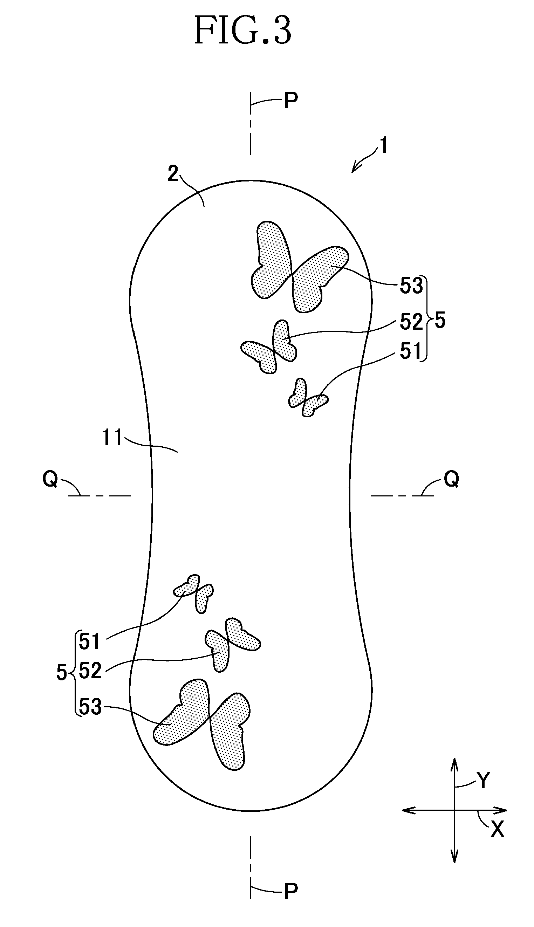

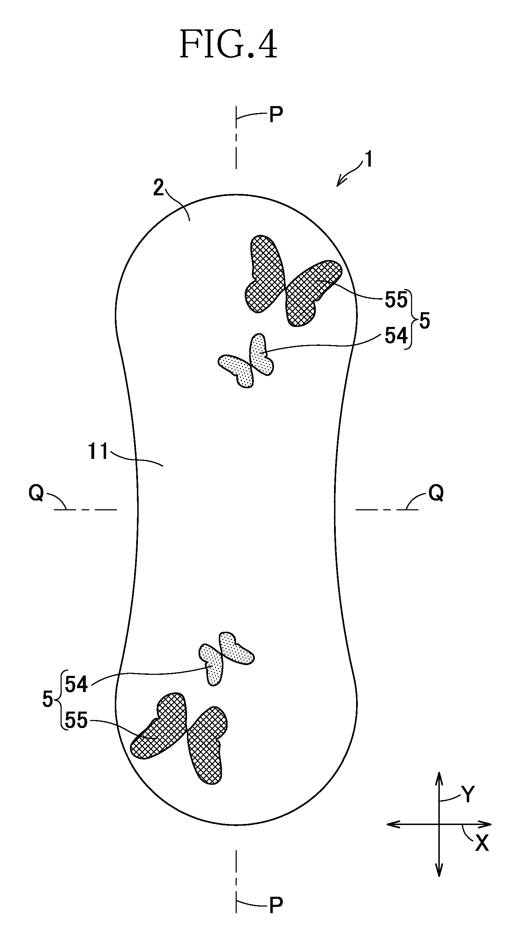

{Fig. 1} Fig. 1 is a plan view of a panty liner as a first embodiment of the present invention.
{Fig. 2} Fig. 2 is a sectional view taken along the line II-II in Fig. 1.
{Fig. 3} Fig. 3 is a plan view of the panty liner as a second embodiment of the present invention.
{Fig. 4} Fig. 4 is a plan view of a panty liner as a third embodiment of the present invention.

### {Description of Embodiments}

### (First Embodiment)

Now the bodily fluid absorbent article according to the present invention will be described in detail taking a panty liner 1 for an example thereof. Figs 1 and 2 illustrate a first embodiment of the panty liner 1 wherein Fig. 1 is a plan view of the panty liner 1 and Fig. 2 is a sectional view taken along the line II-II in Fig. 1. The panty liner 1 has a longitudinal direction Y, a transverse direction X, an imaginary longitudinal center line P-P bisecting a width dimension of the panty liner 1 in the transverse direction X and an imaginary transverse center line Q-Q bisecting a dimension of the panty liner 1 in the longitudinal direction Y and is formed almost symmetrically about these imaginary longitudinal and transverse center lines P-P, Q-Q.

The panty liner 1 includes a liquid-pervious topsheet 2 lying on a skin-facing side, a liquid-impervious backsheet 3 lying on a garment-facing side and a second sheet 4 sandwiched between the top- and backsheets 2, 3 so as to define an intermediate layer. The topsheet 2 may be formed, for example, of an air-through fibrous nonwoven fabric or a spun laced fibrous nonwoven fabric made of hydrophilic fibers having a mass per unit area preferably in a range of about 10 to about 100g/m², more preferably in a range of about 20 to about 50g/m² and even more preferably of about 30g/m². The second sheet 4 may be formed, for example, of a liquid-absorbent sheet such as a spun laced fibrous nonwoven fabric, an air laid fibrous nonwoven fabric or a tissue paper each made of hydrophilic fibers such as cotton and having a mass per unit area preferably in a range of about 14 to about 100g/m², more preferably in a range of about 20 to about 50g/m² and even more preferably of about 40g/m². The backsheet 3 may be formed, for example, of a plastic film or a fibrous nonwoven fabric having a mass per unit area preferably in a range of about 10 to 50g/m² and more preferably of about 35g/m². The backsheet 3 is desirably air-permeable.

In the panty liner 1, the respective sheets are uniform in shape as well as in size and have a width dimension in the transverse direction X gradually reduced toward the imaginary transverse center line Q-Q. By shaping the panty liner 1 in this manner, it is possible to prevent the panty liner 1 from being irregularly deformed in the wearer's crotch region and thereby to keep the panty liner 1 in close contact with the wearer's body.

The panty liner 1 as has been described above is formed with display elements 5 adapted to be visually recognized through the topsheet 2. Each of these display elements 5 is defined by a graphic. These display elements 5 are coated with coloring material in a gradation such that the color density gradually increases from the imaginary transverse center line Q-Q towards the outer periphery of the article as viewed in the longitudinal direction Y. In other words, a density of coloring material in each of the display elements gradually increases from the inner zone towards the outer zone thereof. Such gradation of the coloring material density ensures that the average mass of the coloring material per the predetermined area in the display elements 5 gradually increases from the imaginary transverse center line Q-Q towards the periphery of the article as viewed in the longitudinal direction Y. The term "predetermined area" used herein means respective predetermined areas defined by a sub-area extending inwardly and a sub-area extending outwardly as viewed in the longitudinal direction within the same graphic.

As one of the methods to obtain a gradually increasing density of coloring material, the sheet may be coated with coloring material in a striped pattern with the stripe intervals gradually reducing as in the illustrated embodiment. Alternatively, the sheet may be coated with coloring material in a dotted pattern with the dot intervals gradually reducing. In addition, a coating amount of the coloring material may be gradually increased to obtain a desired gradation. No particular method is specified so long as the density of the coloring material may be gradually increased outwardly in the longitudinal direction Y.

The display elements 5 may be formed on the surface 41 of the second sheet 4 facing the topsheet 2, for example, by flexographic printing. As the coloring material, for example, water-based ink may be used. To prevent such coloring material from running off together with bodily fluids such as menstrual blood, resin contained in a vehicle may be polymerized in a step of drying the ink. Polymerization may be carried out by various means such as drying, UV irradiation, electron irradiation and heating.

Color phase, color saturation and color brightness of the coloring material are not specified so long as the display elements 5 may be visually recognized through the topsheet 2. In the white panty liner 1, the coloring material having a color phase other than white, for example, red, blue or yellow may be used. Even when the color phase of coloring material having the same color phase as that of the panty liner 1 is used, the display elements may be visually recognizable by differentiating the color saturation and/or the color brightness from that or those of the panty liner 1. It is also possible to configure the display elements 5 so that the display elements 5 may not be visually recognized unless absorption of menstrual blood occurs and the white graphics become visually recognizable upon absorption of menstrual blood. For example, it is possible to use a white coloring material so that the white graphics may emerge or change from white to the other color upon absorption of menstrual blood. Furthermore, each of the display elements 5 may include visually unrecognizable portions so long as the display element 5 as a whole is visually recognizable. Specifically, the function of the display elements according to the present invention may be obtained even when each of the display elements 5 is formed by partially using the same color as the panty liner 1, i.e., the visually unrecognizable color so long as the display elements 5 are visually recognizable in their entirety.

The coloring material density of the display elements 5 may be adjusted to be relatively low on the inside and relatively high on the outside as viewed in the longitudinal direction Y to ensure that the mass of the coloring material may be smaller in the vicinity of the middle region 11 defined about the imaginary transverse center line Q-Q than the mass of the coloring material on the outside thereof. In a dried condition, the coloring material defining the display elements 5 becomes liquid-impervious and will deteriorate liquid-perviousness of the sheet coated with coloring material as the density of the coloring material increases. However, at least in the middle region of the sheet coated with the coloring material, in the present invention, the liquid-perviousness of this sheet would not be deteriorated due to the presence of the color material since the mass of the coloring material is gradually reduced from the outside toward the inside of the article. Considering that the bodily fluid absorbent article such as the panty liner 1 is usually put on the wearer's body so that the middle region 11 thereof may be aligned with the wearer's excreting organ, it is essential to prevent the liquid-perviousness of the middle region 11 from being deteriorated.

By gradually increasing the mass of coloring material from the inside towards the outside as viewed in the longitudinal direction Y, the liquid-perviousness of the sheet is appropriately restricted on the outside region of the sheet to prevent bodily fluids from running outward. Furthermore, in the region of the sheet coated with the coloring material, bodily fluids absorbed on the side under the layer of the coloring material can be effectively hidden so that such bodily fluids are not visually recognizable through the side of the top sheet 2.

In the middle region 11, a non-display region having none of the display elements 5 is defined. The non-display region extends around the intersection of the imaginary longitudinal center line P-P and the imaginary transverse center line Q-Q and preferably has a width dimension of about 20mm or larger in the transverse direction X and a dimension of about 30mm or longer in the longitudinal direction Y. The middle region 11 is preferably adapted to be put in direct contact with the wearer's excreting organ and kept in close contact with the wearer's body. If such middle region 11 is formed with the display elements, the wearer's skin might be soiled with the coloring material. However, the middle region 11 is the non-display region according to this embodiment and therefore the wearer is free from such apprehension.

The display elements 5 are located symmetrically about the intersection of the imaginary longitudinal center line P-P and the imaginary transverse center line Q-Q. By locating the display elements 5 in such point symmetric relationship, the middle region is free from the presence of the display elements and creates aesthetic and neat impression.

While the display elements 5 are formed on the surface 41 of the second sheet 4 by printing according to the present embodiment, it is possible to form them on the skin-facing side 21 of the topsheet 2 or the garment-facing side 22 of the topsheet 2 which is facing the second sheet 4. When the display elements 5 are formed on the skin-facing side 21 of the topsheet 2, any suitable measures are preferably taken to prevent the wearer's skin from being soiled with ink. When the display elements 5 are formed on the second sheet 4, a total luminous reflectance is preferably adjusted so that the display elements 5 may be visually recognized through the topsheet 2.

### (Second Embodiment)

Fig. 3 is a view similar to Fig. 1, illustrating a second embodiment. This second embodiment is characterized by a plurality of graphics 51, 52, 53 having different sizes as the display elements 5. The other aspects are similar to those of the first embodiment. The components similar to those of the first embodiment are designated by the similar reference numerals and will not be repetitively described in detail.

The graphics 51, 52, 53 are arranged to have respective areas gradually enlarged from the imaginary transverse center line Q-Q outwardly as viewed in the longitudinal direction Y. Specifically, the graphic 51 lying closest to the imaginary transverse center line Q-Q has the smallest area, the graphic 52 lying immediately outside the graphic 51 has an area smaller than that of the graphic 51 and the graphic 53 lying outside the graphic 52 has an area larger than that of the graphic 52. In the graphics 51, 52, 53, a density of the coloring material is substantially uniform. However, the sizes of the respective graphics are gradually enlarged as viewed outwardly in the longitudinal direction Y so that, in the panty liner 1 as a whole, the mass of coloring material per the predetermined area gradually increases outwardly as viewed in the longitudinal direction Y. The term "predetermined area" used herein means (a full width dimension of the panty liner 1 in the transverse direction X) x (a predetermined dimension of the panty liner 1 in the longitudinal direction Y) and the average mass of the coloring material in this predetermined area gradually increases outwardly as viewed in the longitudinal direction Y.

The sizes of the respective graphics may be differentiated to increase the mass of coloring material in gradation more easily than by differentiating the coating coloring material or differentiating an interval between each pair of the adjacent coating line of the coloring material. While the density of the coloring material in the respective graphics 51, 52, 53 is uniform according to this embodiment, it is possible to differentiate the density of coloring material in the respective graphics one from another. In this way, a gradation of the mass among these graphics 51, 52, 53 can be emphasized.

### (Third Embodiment)

Fig. 4 is a view similar to Fig. 1, illustrating a third embodiment. This third embodiment is characterized by a plurality of graphics 54, 55 having different sizes as the display elements 5. The other aspects are similar to those of the first embodiment. The components similar to those of the first embodiment are designated by the same reference numerals and will not be repetitively described in detail.

The graphic 55 is located lateral to the graphic 54 in the longitudinal direction Y as well as in the transverse direction X wherein the graphic 55 has an area larger than that of the graphic 54 and a coating amount of the coloring material in the graphic 55 is larger than that in the graphic 54.

In the way as has been described above, the mass of the coloring material per the predetermined area may be gradually increased outwardly in the longitudinal direction Y as well as in the transverse direction X. Both the size of the graphics and the amount of the coloring material may be differentiated to emphasize the gradation defining the mass of the coloring material.

While two graphics or two sets of graphics are located symmetrically about the intersection of the imaginary longitudinal center line P-P and the imaginary transverse center line Q-Q in the first and second embodiments, the present invention is not limited to such particular arrangement and it is possible to form the graphics on any one of these two locations. The present invention is not limited to such point symmetric location of the graphics but it is also possible to locate the graphics in an asymmetric arrangement.

While a flexographic printing has been exemplarily described above as means for forming the display elements 5, the other printing methods such as a gravure printing, an ink jet printing and a screen printing may be selectively used to form the display elements 5. Regarding the types of ink for such printing, not only water-based inks and oil-based inks, but also UV inks may be used. Printing methods suitable for respective types of ink may be selectively used and pigment and vehicles commonly used in the related technical field may be used.

The topsheet 2, the second sheet 4 and the backsheet 3 are not limited to those used in this embodiment but may be selected from other various types of sheets. While a liquid-absorbent single sheet is used as the second sheet 4 in this embodiment, it is possible to layer two or more sheets to form the second sheet 4. It is also possible to use a liquid-pervious sheet as the second sheet 4. As the liquid-pervious sheet, for example, a hydrophilic air-through nonwoven fabric having a mass per unit area in a range of about 10 to about 100g/m², preferably in a range of about 20 to 50g/m² and more preferably of about 30g/m² may be used. It is also possible to use a laminate of liquid-pervious sheet and liquid-absorbent sheet as the second sheet 4. These sheets 2, 4, 3 are bonded together on surfaces thereof facing one another by the intermediary of bonding means such as hot melt adhesive.

While the embodiments in which the second sheet 4 is used as the intermediate layer have been described above, the present invention is not limited to this but, for example, an absorber may be also used as the intermediate layer. As the absorber, compression molded fluff pulp fibers may be used and such fluff pulp fibers may be directly sandwiched between the topsheet 2 and the backsheet 3 to obtain the bodily fluid absorbent article. In this case, the display elements 5 are desirably formed on the topsheet 2. It is also possible to use fluff pulp and/or super-absorbent polymer particles wrapped with tissue paper as the absorber.

While the dimension of the panty liner 1 in the longitudinal direction Y is assumed in a range of about 90 to about 200mm in the first and second embodiments, the present invention is not limited to such range and may be set to other various dimensions. When the dimension of the panty liner 1 in the longitudinal direction is set to a relatively large value, a dimension of each display element 5 in the longitudinal direction Y is preferably set to a relatively small dimension so that the correspondingly large non-display region extending in the middle zone 11 may be ensured. Such dimensional relationship, particularly in the panty liner 1 dimensioned to be relatively large in the longitudinal direction Y, magnifies the dimension in the longitudinal direction Y thereof and thereby provides the wearer with a sense of reassurance.

For the display elements, various graphics, for example, snow crystal marks or floral motifs may be used. The bodily fluid absorbent article is not limited to the panty liner and may be implemented in other forms such as menstruation napkins and urine absorbent pads.

### {Reference Signs List}

- 1: panty liner (bodily fluid absorbent article)
- 2: topsheet
- 3: backsheet
- 4: second sheet (intermediate layer)
- 5: display elements
- 51: graphic
- 52: graphic
- 53: graphic
- 54: graphic
- 55: graphic
- 11: middle region
- X: transverse direction
- Y: longitudinal direction

## Claims

1. A bodily fluid absorbent article having a longitudinal direction, a transverse direction, a skin-facing side and a garment-facing side and comprising a liquid-pervious topsheet lying on the skin-facing side, a liquid-impervious backsheet lying on the garment-facing side and an intermediate layer sandwiched between the top- and backsheets, wherein:
display elements are formed of coloring material applied to at least one of the topsheet and the intermediate layer so that an average mass of the coloring material per a predetermined area gradually increases outwardly as viewed in the longitudinal direction.

2. The bodily fluid absorbent article according to Claim 1, wherein an average mass of the coloring material per the predetermined area gradually increases from an imaginary longitudinal center line bisecting a width dimension in the transverse direction outwardly as seen in the transverse direction.

3. The bodily fluid absorbent article according to Claim 1 or 2, wherein a non-display region including none of the display elements is defined in a middle region of the article as viewed in the longitudinal direction.

4. The bodily fluid absorbent article according to any one of Claims 1 through 3, wherein the display elements are formed of a plurality of graphics and the graphics lying closer to an outer edge of the article are larger than the graphics lying closer to the middle of the article as viewed in at least one of the longitudinal direction and the transverse direction.

5. The bodily fluid absorbent article according to any one of Claims 1 through 4, wherein a density of the coloring material is higher in the graphics lying closer to an outer edge of the article than in the graphics lying closer to the middle of the article as viewed in at least one of the longitudinal direction and the transverse direction.
